# EUROPÄISCHE PATENTSCHRIFT

(11) **EP 1 615 876 B1**
(45) Veröffentlichungstag und Bekanntmachung des Hinweises auf die Patenterteilung: **04.04.2007**
(21) Anmeldenummer: 04725673.0
(22) Anmeldetag: 05.04.2004
(51) Int. Cl.: C07C 231/24, C07C 233/05

(54) **VERFAHREN ZUR REINIGUNG VON DIMETHYLACETAMID (DMAC)**
METHOD FOR THE PURIFICATION OF DIMETHYLACETAMIDE (DMAC)
PROCEDE POUR PURIFIER DU DIMETHYLACETAMIDE

(30) Priorität: 03.04.2003 DE 10315214
(43) Veröffentlichungstag der Anmeldung: 18.01.2006
(73) Patentinhaber: BASF Aktiengesellschaft, 67056 Ludwigshafen (DE)
(72) Erfinder: PESCHEL, Werner, 67251 Freinsheim (DE); SCHOENMAKERS, Hartmut, 69121 Heidelberg (DE); ALTHAUS, Klaus, 68163 Mannheim (DE); KIRCHNER, Tanja, 66629 Freisen (DE); STAATZ, Hartmut, 69124 Heidelberg (DE)
(74) Vertreter: Isenbruck, Günter
(86) Internationale Anmeldenummer: PCT/EP2004/003595
(87) Internationale Veröffentlichungsnummer: WO 2004/087639

(56) Entgegenhaltungen:
- WO-A-20/04002926
- US-A- 3 557 207
- US-A- 3 687 820

## Beschreibung

Die Erfindung betrifft ein Verfahren zur destillativen Reinigung von Roh-Dimethylacetamid. Für Dimethylacetamid wird im Folgenden die abgekürzte Bezeichnung DMAc verwendet.

DMAc wird überwiegend als Lösungsmittel eingesetzt, beispielsweise als Lösungsmittel zum Lösungsspinnen von elastischen Polyurethan-Blockcopolymeren, die unter dem Markennamen Spandex^{®} oder Lycra^{®} bekannt sind, wie auch zur Herstellung von Hohlfasern.

Um beim Lösungsspinnen qualitativ hochwertige Fasern erhalten zu können, werden an das hierbei eingesetzte DMAc die nachfolgend aufgeführten Spezifikationenanforderungen gestellt: Wassergehalt < 100 ppm, pH-Wert zwischen 6,5 und 7 und spezifische elektrische Leitfähigkeit < 0,6 µS/cm, oder auch < als 0,2 µS/cm. Die elektrische Leitfähigkeit des Rein-DMAc wird im Wesentlichen durch seinen Gehalt an Verunreinigungen, wie Säuren, insbesondere Essigsäure und Salzen, insbesondere den Aminsalzen der Essigsäure, bewirkt. Die angegebenen Spezifikationen des Rein-DMAc bezüglich pH-Wert und elektrischer Leitfähigkeit entsprechen einem Gehalt an Essigsäure von weniger als 50 Gew.-ppm.

Ein DMAc, das diese Spezifilcationsanforderungen erfüllt, wird im Folgenden als Rein-DMAc bezeichnet.

Demgegenüber wird als Roh-DMAc ein Gemisch, enthaltend DMAc, bezeichnet, das die oben definierten Spezifikationsanforderungen nicht erfüllt. Roh-DMAc enthält als Hauptkomponenten neben DMAc Amin, Essigsäure und Wasser. Hierbei ist der Essigsäure-Gehalt von erfindungsgemäß zu reinigendem Roh-DMAc nicht größer als 20 Gew.-%.

Als wasserhaltiges Roh-DMAc wird vorliegend ein Roh-DMAc und einem Wassergehalt zwischen 1 und 99 Gew.-%, oder auch zwischen 2 und 99 Gew.-%, auch zwischen 80 und 98 Gew.-%, insbesondere zwischen 95 und 98 Gew.-%, oder auch zwischen 1 und 6 Gew.-% verstanden.

Bekannte Anlagen zur destillativen Reinigung von DMAc werden unter Vakuum betrieben, um Zersetzungsreaktionen des DMAc wirksam zu unterdrücken und dadurch die Einhaltung der Spezifikationsanfbrderungen zu gewährleisten.

Es wurde jedoch gefunden, dass in Destillationsanlagen, die wie bislang üblich unter Vakuum betrieben wurden, die Spezifikationsanforderungen häufig nicht erfüllt werden können.

US-A-3 557 207 und US-A-3 687 820 beschreiben die destillative Trennung von DMAc und Essigsäure.

Die FR-PS 1,406,279 beschreibt ein Verfahren zur Herstellung von DMAc durch Umsetzung von Essigsäure mit Dimethylamin und destillative Auftrennung des Reaktionsgemisches in einer Zweikolonnen-Anordnung, wobei die erste Kolonne bei Atmosphärendruck oder geringfügig erhöhtem Druck, einer Sumpftemperatur zwischen 165 und 170°C, einer Kopftemperatur zwischen 95 und 105°C und einer Temperatur zwischen 100 und 200°C im Bereich des Kolonnen-Feeds, der etwa in der Mitte der Kolonne angeordnet ist, betrieben wird. Aus der ersten Kolonne wird ein Kopfstrom, enthaltend Dimethylamin und Wasser abgezogen und ein gasförmiger oder flüssiger Sumpfstrom, enthaltend DMAc, Essigsäure sowie Monomethylacetamid. Dieser Sumpfstrom wird einer zweiten Kolonne, etwa im mittleren Bereich derselben, zugeführt und in einen Kopfstrom, enthaltend gereinigtes DMAc und einen Sumpfstrom, enthaltend das ternäre Azeotrop von Essigsäure, DMAc und Monomethylacetamid, aufgetrennt.

Zur Auslegung und zu den Betriebsbedingungen für die zweite Kolonne wird lediglich angegeben, dass es sich um einen klassischen Kolonnentyp und klassische Betriebsbedingungen handelt. Aus den Beispielen ist zu entnehmen, dass die zweite Kolonne bei einem Druck von 400 mg Hg und einer Kopftemperatur von 143°C betrieben wird, sowie dass das gereinigte DMAc noch etwa 225 ppm Essigsäure und ebensoviel Wasser enthält, und somit den hohen Spezifikationsanforderungen, wie sie für Rein-DMAc für die Zwecke der vorliegenden Erfindung definiert sind, nicht erfüllen.

Es war daher Aufgabe der Erfindung, ein Verfahren zur destillativen Reinigung von Roh-DMAc zur Verfügung zu stellen, das die Einhaltung der geforderten Spezifikationen insbesondere für das Lösungsspinnen zur Herstellung von elastischen Polyurethan-Blockcopolymerfasern sowie zur Herstellung von Hohlfasern gewährleistet.

Darüber hinaus sollte das Verfahren die Abtrennung des Wassers aus dem Roh-DMAc in einem Reinheitsgrad, insbesondere mit einem Restgehalt an DMAc von unter 50 ppm gewährleisten, der die Wiederverwendung oder die problemlose Entsorgung desselben als Abwasser erlaubt.

Die Lösung geht aus von einem Verfahren zur destillativen Reinigung von Roh-Dimethylacetamid (Roh-DMAc), enthaltend DMAc, Leichtsieder und Schwersieder, wobei man die Leichtsieder und die Schwersieder unter Erhalt von Rein-DMAc in einer der nachfolgend aufgeführten Kolonnenkonfigurationen:
(I) eine Hauptkolonne mit Seitenabzugslcolonne oder
(II) eine Trennwandkolonne
abtrennt.

Die Erfindung ist dadurch gekennzeichnet, dass man in der Kolonnenkonfiguration (I) zumindest die Hauptkolonne, und in der Kolonnenkonfiguration (II) die Trennwandkolonne mit einem Kopfdruck im Bereich von 0,5 bis 1,8 bar absolut betreibt.

Es wurde überraschend gefunden, dass durch eine Betriebsweise bei Normaldruck oder bei einem Druck, der geringfügig oberhalb oder unterhalb des Normaldrucks liegt, der Reinheitsgrad des durch Destillation gewonnen DMAc wesentlich besser ist als bei bekannten Verfahren, die, um Verunreinigungen durch Zersetzung oder Nebenreaktionen zu vermeiden, unter Vakuum und somit bei wesentlich niedrigeren Temperaturen betrieben werden.

Demgegenüber haben die Erfinder überraschend gefunden, dass gerade eine Druck- und somit eine Temperaturerhöhung für die Erzielung eines Reinproduktes mit einem niedrigeren Anteil an Verunreinigungen erforderlich ist.

DMAc hat einen Siedepunkt bei Normaldruck von 166°C.

Als Leichtsieder werden vorliegend Substanzen bezeichnet, deren Siedepunkt unterhalb des Siedepunktes von DMAc liegt und als Schwersieder Substanzen, deren Siedepunkt oberhalb des Siedepunktes von DMAc mit Essigsäure liegt.

Leichtsieder sind im vorliegenden Verfahren insbesondere Wasser, daneben Dimethylamin und Diethylamin.

Schwersieder sind insbesondere das Azeotrop von DMAc mit Essigsäure, daneben Ethanolamine, wie auch sogenannte Heavyends, d.h. hochmolekulare Zersetzungsprodukte in Form von hochviskosen Flüssigkeiten oder Feststoffen.

Als Kolonne in Abtriebsfahrweise wird eine Kolonne bezeichnet, die nur unterhalb der Zufuhrung des in der Kolonne aufzutrelillenden Gemisches trennwirksame Einbauten aufweist. Demgegenüber weist eine Kolonne in Verstärkungsfahrweise nur oberhalb der Zuführung des in der Kolonne aufzutrennenden Gemisches trennwirksame Einbauten auf

Als Trennwandkolonne wird in bekannter Weise eine Kolonne mit in Kolonnenlängsrichtung angeordneter Trennwand bezeichnet, die in Teilbereichen der Kolonne eine Vermischung von Flüssigkeits- und Brüdenströmen verhindert. Die Trennwand teilt den Kolonneninnenraum in bekannter Weise in einen Zuführbereich, einen Entnahmebereich, einen oberen gemeinsamen Kolonnenbereich sowie einen unteren gemeinsamen Kolonnenbereich.

Bei der vorliegenden Trennaufgabe müssen aus seinem Ausgangsgemisch Leichtsieder und Schwersieder abgetrennt werden und das Wertprodukt, Rein-DMAc, als Mittelsieder gewonnen werden.

Diese Trennaufgabe kann insbesondere mit einer der nachfolgend aufgeführten Kolormenkonfigurationen:
(I) eine Hauptkolonne mit Seitenabzugskolonne oder
(II) eine Tremiwandkolonne
gelöst werden.

Erfindungsgemäß muss in jeder der vorstehend aufgeführten Kolonnenkonfigurationen zumindest die Kolonne, in der der Leichtsieder Wasser noch nicht weitgehend abgetrennt ist, bei einem Druck im Bereich von 0,5 bis 1,8 bar absolut, d.h. bei Normaldruck oder bei gegenüber Normaldruck geringfügig erniedrigtem oder erhöhtem Druck betrieben werden.

Bevorzugt liegt der Kopfdruck in den oben genannten Kolonnen im Bereich von 0,8 bis 1,5 bar absolut, besonders bevorzugt im Bereich von 1,0 bis 1,3 bar absolut.

Es ist auch möglich, dass man auch die Seitenabzugskolonne in der Kolonnenkonfiguration (I) mit einem Kopfdruck im Bereich von 0,5 bis 1,8 bar absolut, bevorzugt von 0,8 bis 1,5 bar absolut, besonders bevorzugt von 1,0 bis 1,3 bar absolut, betreibt.

Der Roh-DMAc-Strom kann sowohl gasförmig als auch flüssig zugeführt werden, wobei die Zuführung in flüssigem Aggregatzustand aus energetischen Gründen vorteilhaft ist.

In allen Kolonnenkonfigurationen können als trennwirksame Einbauten grundsätzlich alle bekannten Einbauten, insbesondere Böden, Füllkörper oder Packungen eingesetzt werden.

Als Einbauten eignen sich übliche Einbauten wie handelsübliche Böden, Füllkörper oder Packungen, beispielsweise Glockenböden, Tunnelböden, Ventilböden, Siebböden, Dualflowböden und Gitterböden, Pall-Ringe^{®}, Berl^{®}-Sattelkörper, Netzdrahtringe, Raschig-Ringe^{®}, Intalox^{®}-Sättel, Interpak^{®}-Füllkörper und Intos^{®}, aber auch geordnete Packungen, wie beispielsweise Sulzer-Mellapak^{®}, Sulzer-Optiflow^{®} Kühni-Rombopak^{®} und Montz-Pa^{®} sowie Gewebepackungen. Bevorzugt sind Hochleistungspackungen.

Bevorzugt werden unterhalb der Zuführung des zu reinigenden Roh-DMAc-Stromes trennwirksame Einbauten mit hohen Verweilzeiten, insbesondere Böden, eingesetzt, d.h. in der Kolonnenkonfiguration (I) im Abtriebsteil der Hauptkolonne und in der Kolonnenkonfiguration (II) im Abtriebsteil der Trennwandkolonne. Durch die Einbauten mit höheren Verweilzeiten wird eine verstärkte Rückspaltung des Essigsäure-Diethylamin-Addulctes gewährleistet.

Der Durchmesser der Kolonnen richtet sich nach den jeweils angestrebten Durchsätzen und kann nach den gängigen Regeln der Technik vom Fachmann leicht ermittelt werden.

Die Auslegung der Kolonnen bezüglich Höhe und Positionierung von Zu- und Abläufen kann nach dem bekannten Konzept der theoretischen Trennstufen in Verbindung mit den gewählten Einbauten ermittelt werden.

Als eine theoretische Trennstufe wird in bekannter Weise diejenige Kolonneneinheit verstanden, welche ein Anreichern der leichter flüchtigen Komponente entsprechend dem thermodynamischen Gleichgewicht bewirkt, vorausgesetzt, dass ideale Durchmischung vorliegt, flüssige und gasförmige Phase im Gleichgewicht stehen und kein Mitreißen von Flüssigkeitstropfen erfolgt (vgl. Vauck, Müller: Grundoperationen chemischer Verfahrenstechnik, VCH-Verlagesellschaft mbH, Weinheim, 1988).

Die Zahl der theoretischen Trennstufen für den Bereich oberhalb der Zuführung des zu reinigenden Roh-DMAc-Stromes, d.h. für den oberen Bereich der Hauptkolonne in der Kolormenkonfiguration (I) oder für den Bereich oberhalb des oberen Endes der Trennwand in der Trennwandkolonne wird nach üblichen verfahrenstechnischen Überlegungen in Abhängigkeit vom Leichtsiederanteil im Roh-DMAc und dem zulässigen Verlust an DMAc über Kopf der ersten Kolonne festgelegt.

Die Zahl der theoretischen Trennstufen im Abtriebsteil der Hauptkolonne in der Kolonnenkonfiguration (I) oder der Trennwandkolonne unterhalb des Trennblechs in der Kolonnenkonfiguration (II) wird vorzugsweise im Bereich von 5 bis 30, insbesondere im Bereich von 10 bis 25, besonders bevorzugt im Bereich von 12 bis 18 festgelegt.

Bevorzugt sind die Hauptkolonne der Kolonnenkonfiguration (I) oder die Trennwandkolonne jeweils mit einem Sumpfverdampfer und einem Kondensator am Kolonnenkopf ausgestattet.

Vorteilhaft stellt man die Temperatur am Kopf der Hauptkolonne beziehungsweise der Trennwandkolonne im Bereich von 70 bis 130°C, bevorzugt im Bereich von 85 bis 115°C, besonders bevorzugt im Bereich von 95 bis 105°C, und die Temperaturen im Sumpf der Hauptkolonne beziehungsweise der Trennwandkolonne jeweils im Bereich von 150 bis 200°C, bevorzugt im Bereich von 160 bis 190°C, besonders bevorzugt im Bereich von 170 bis 180°C, ein.

Die destillative Reinigung von Roh-DMAc führt man vorteilhaft in einer Kolonnenkonfiguration (I) durch, deren Hauptkolonne einen gasförmigen Seitenabzug aufweist und deren Seitenabzugskolonne in Verstärkungsfarweise betrieben wird.

Bevorzugt weist die Hauptkolonne oberhalb des gasförmigen Seitenabzugs einen geringeren Durchmesser gegenüber dem Bereich der Hauptkolonne unterhalb des gasförmigen Seitenabzugs auf.

Weiter kann die destillative Reinigung in einer Kolonnenkonfiguration (I) durchgerührt werden, in der die Hauptkolonne einen flüssigen Seitenabzug aufweist und die Seitenabzugskolonne in Abtriebsfahrweise betrieben wird.

Die Seitenabzugskolonne aus der Kolonnenkonfiguration (I) unterscheidet sich von klassischen Destillationskolonnen, wie sie beispielsweise einer Zwei-Kolonnen-Fahrweise nach dem Stand der Technik eingesetzt werden unter anderem dadurch, dass sie keinen eigenen Sumpfverdampfer hat und bezüglich ihrer Trennstufenzahl kleiner dimensioniert ist. Im übrigen kann die Seitenabzugskolonne entsprechend und abhängig von der Zusammensetzung des ihr zugeführten Seitenabzugstromes aus der Hauptkolonne vom Fachmann anhand weniger Routineversuche und -berechnungen ausgelegt werden. Die Seitenabzugskolonne kann auch die üblichen Kolonneneinbauten enthalten, wobei Packungen besonders bevorzugt sind.

Der Schwersiederaustrag der genannten Kolonnenkonfigurationen (I) oder (II) enthält neben Essigsäure auch das Wertprodukt DMAc, da Essigsäure und DMAc ein Schwersiederazeotrop ausbilden. Die vollständige thermische Auftrennung von Essigsäure und DMAc ist deshalb destillativ nicht möglich. Das im Sumpf der Hauptkolonne beziehungsweise der Trennwandkolonne verbleibende Schwersiederazeotrop aus Essigsäure und DMAc wird ausgeschleust und kann energetisch vorteilhaft zur Rückgewinnung des darin enthaltenen DMAc mit Lauge neutralisiert und das DMAc durch Eindampfen des Neutralisats z.B. in einen Fallfilinverdampfer oder Wirkblattverdampfer gegebenenfalls bei vermindertem Druck zurückgewonnen werden. Die Neutralisation und das anschließende Eindampfen können sowohl kontinuierlich als auch diskontinuierlich erfolgen.

Vorteilhaft wird die destillative Reinigung kontinuierlich betrieben.

Durch das erfindungsgemäße Verfahren wird somit in überraschend einfacher Weise ein Rein-DMAc gewonnen, das die geforderten Spezifikationen bezüglich Wassergehalt, pH-Wert und elektrischer Leitfähigkeit erfüllt, durch Destillation bei Normaldruck oder bei gegenüber Normaldruck geringfügig erniedrigtem oder erhöhtem Druck. Die Betriebsweise bei oder nahezu bei Normaldruck ist gegenüber einer Betriebsweise unter Vakuum, wie sie im Stand der Technik für die Reinigung von DMAc bislang erforderlich schien, wesentlich wirtschaftlicher, insbesondere bezüglich Investitions- und Betriebskosten. Darüber hinaus erhöht sich mit dem Anstieg des Betriebsdrucks die Kapazität der Apparate, da sich bei gleich bleibendem Gasmengenstrom die Gasgeschwindigkeit aufgrund der höheren Gasdichte verringert.

Die Erfindung wird im Folgenden anhand einer Zeichnung und eines Ausführungsbeispiels näher erläutert.

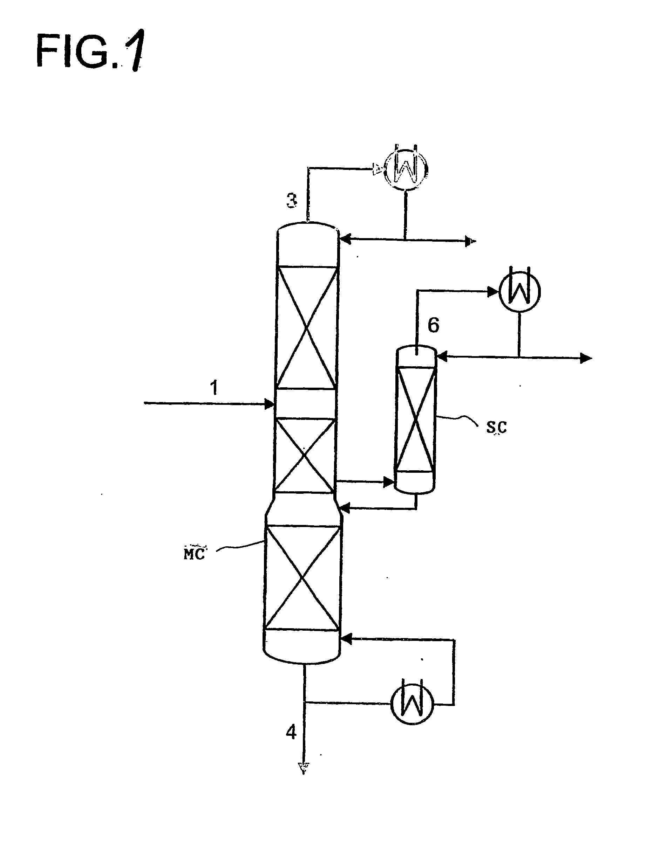

- Figur 1: zeigt ein Verfahrensschema für eine kolonnenkonfiguration (I).

Figur 1 zeigt ein bevorzugtes Ausführungsbeispiel des erfindungsgemäßen Verfahrens, wobei eine Kolonnenkonfiguration (I) mit Hauptkolonne HK und Seitenabzugskolonne SK gewählt wird.
Die Hauptkolonne HK wird im mittleren Bereich derselben ein Roh-DMAc-Strom 1 zugeführt. Am Kolonnenkopf wird ein Leichtsieder enthaltender Strom 3 abgezogen und aus dem Kolonnensumpf ein Schwersieder enthaltender Strom 4. Über einen Seitenabzug wird ein gasförmiger Strom abgezogen, der noch spezifikationsschädliche schwersiedende Verunreinigungen, insbesondere Essigsäure, enthält, und der in der Seitenabzugskolonne SK, die in Verstärkungsfahrweise betrieben wird, gereinigt wird unter Erhalt eines Rein-DMAc-Stromes 6 am Kolonnenkopf.

### Ausführungsbeispiel

In einer Kolonnenkonfiguration (I) entsprechend der schematischen Darstellung in Figur 1 wurde ein flüssiger Roh-DMAc-Strom 1 mit folgenden Komponenten:
DMAc 99,2 Gew.-%, Dimethylamin 6 ppm, DMF 0,2 Gew.-%, Wasser 0,3 Gew.-%, Essigsäure 100 ppm, Ameisensäure 350 ppm, Phosphorsäure 50 ppm und Heavyends 0,2 Gew.-% bei einer Temperatur von 20°C auf die 30igste theoretische Trennstufe einer Hauptkolonne mit 57 theoretischen Trennstufen, bei Zählung der Trennstufen von unten nach oben, zugeführt.

Von der 9. theoretischen Trennstufe wurde ein gasförmiger Seitenstrom bei einer Temperatur von 171,9°C, mit folgender Zusammensetzung abgezogen:
DMAc 99,9 Gew.-%, DMF 720 ppm und Essigsäure 280 ppm.

Die spezifikationsschädliche Konzentration an Essigsäure von 280 ppm konnte in der Seitenabzugskolonne auf spezifikationsunschädliche 20 ppm im Kopfstrom 6, der als Rein-DMAc-Strom abgezogen wird, reduziert werden. Die Hauptkolonne HK und die Seitenkolonne SK wurden bei Normaldruck betrieben.

## Patentansprüche

1. Verfahren zur destillativen Reinigung von wasserhaltigem Roh-Dimethylacetamid (Roh-DMAc), enthaltend DMAc, Leichtsieder und Schwersieder, wobei man die Leichtsieder und die Schwersieder unter Erhalt von Rem-DMAc in einer der nachfolgend aufgeführten Kolonnenkonfigurationen:
(I) eine Hauptkolonne (HK) mit Seitenabzugskolonne (SK) oder
(II) eine Trennwandkotonne (TWK)
abtrennt, **dadurch gekennzeichnet, dass** man in der Kolonnenkonfiguration (I) zumindest die Hauptkolonne (HK), und in der Kolonnenkonfiguration (II) die Trennwandkolonne (TWh) mit einem Kopfdruck im Bereich von 0,5 bis 1,8 bar absolut betreibt.

2. Verfahren nach Anspruch 1, **dadurch gekennzeichnet, dass** man in der Kolonnenkonfiguration (I) zumindest die Hauptkolonne (HK) und in der Kolonnenkonfiguration (II) die Trennwandkolonne (TWK) bei einem Kopfdruck im Bereich von 0,8 bis 1,5, bevorzugt im Bereich von 1,0 bis 1,3 bar absolut betreibt.

3. Verfahren nach Anspruch 1 oder 2, **dadurch gekennzeichnet, dass** man die Seitenabzugskolonne (SK) in der Kolonnenkonfiguration (I) mit einem Kopfdruck im Bereich von 0,5 bis 1,8 bar absolut, bevorzugt von 0,8 bis 1,5 bar absolut, besonders bevorzugt von 1,0 bis 1,3 bar absolut, betreibt.

4. Verfahren nach einem der Ansprüche 1 bis 3, **dadurch gekennzeichnet, dass** man im Abtriebsteil der Hauptkolonne (HK) in der Kolonnenkonfiguration (I) beziehungsweise im Abtriebsteil der Trennwandkolonne (TWK) in der Kolonnenkonfiguration (II) trennwirksame Einbauten mit hohen Verweilzeiten, bevorzugt Böden, einbaut.

5. Verfahren nach einem der Ansprüche 1 bis 4, **dadurch gekennzeichnet, dass** man im Abtriebsteil der Hauptkolonne (HK) oder der Trennwandkolonne (TWK) 5 bis 30, bevorzugt 10 bis 25, besonders bevorzugt 12 bis 18 theoretische Trennstufen vorsieht.

6. Verfahren nach einem der Ansprüche 1 bis 5, **dadurch gekennzeichnet, dass** man die Hauptkolonne (HK) oder die Trennwandkolonne (TWK) jeweils mit einem Sumpfverdampfer und einem Kondensator am Kolonnenkopf ausstattet.

7. Verfahren nach einem der Ansprüche 1 bis 6, **dadurch gekennzeichnet, dass** man die Temperatur am Kopf der Hauptkolonne (HK) oder der Trennwandkolonne (TWK) im Bereich von 70 bis 130°C, bevorzugt im Bereich von 85 bis 115°C, besonders bevorzugt im Bereich von 95 bis 105°C, die Temperaturen im Sumpf der Hauptkolonne (HK) oder der Trennwandkolonne (TWK) jeweils im Bereich von 150 bis 200°C, bevorzugt im Bereich von 160 bis 190°C, besonders bevorzugt im Bereich von 170 bis 180°C, einstellt.

8. Verfahren nach einem der Ansprüche 1 bis 7, **dadurch gekennzeichnet, dass** man die destillative Reinigung von Roh-DMAc in einer Kolonnenkonfiguration (I) durchführt, deren Hauptkolonne (HK) einen gasförmigen Seitenabzug aufweist und deren Seitenabzugskolonne (SK) in Verstärkungsfahrweise betrieben wird.

9. Verfahren nach Anspruch 8, **dadurch gekennzeichnet, dass** die Hauptkolonne (HK) oberhalb des gasförmigen Seitenabzugs einen geringeren Durchmesser gegenüber dem Bereich der Hauptkolonne (HK) unterhalb des gasförmigen Seitenabzugs aufweist.

10. Verfahren nach einem der Ansprüche 1 bis 7, wobei die destillative Reinigung in einer Kolonnenkonfiguration (I) durchgeführt wird, in der die Hauptkolonne (HK) einen flüssigen Seitenabzug aufweist und die Seitenabzugskolonne (SK) in Abtriebsfahrweise betrieben wird.

11. Verfahren nach einem der Ansprüche 1 bis 10, **dadurch gekennzeichnet, dass** man es kontinuierlich betreibt.

## Claims

1. A process for distillatively purifying aqueous crude dimethylacetamide (crude DMAc) comprising DMAc, low boilers and high boilers by removing the low boilers and the high boilers to obtain pure DMAc in one of the column configurations listed hereinbelow:
(I) a main column (MC) with sidestream column (SC) or
(II) a dividing wall column (DWC),
which comprises operating at least the main column (MC) in column configuration (I) and the dividing wall column (DWC) in column configuration (II) at a top pressure in the range from 0.5 to 1.8 bar absolute.

2. The process according to claim 1, wherein at least the main column (MC) in column configuration (I) and the dividing wall column (DWC) in column configuration (II) are operated at a top pressure in the range from 0.8 to 1.5 bar absolute, preferably in the range from 1.0 to 1.3 bar absolute.

3. The process according to claim 1 or 2, wherein the sidestream column (SC) in column configuration (I) are operated at a top pressure in the range from 0.5 to 1.8 bar absolute, preferably from 0.8 to 1.5 bar absolute, more preferably from 1.0 to 1.3 bar absolute.

4. The process according to any of claims 1 to 3, wherein separating internals having long delay times, preferably trays, are installed in the stripping section of the main column (MC) in column configuration (I) or in the stripping section of the dividing wall column (DWC) in column configuration (II).

5. The process according to any of claims 1 to 4, wherein from 5 to 30, preferably from 10 to 25, more preferably from 12 to 18, theoretical plates are provided in the stripping section of the main column (MC) or of the dividing wall column (DWC).

6. The process according to any of claims 1 to 5, wherein the main column (MC) or the dividing wall column (DWC) are each equipped with a bottom evaporator and a condenser at the top of the column.

7. The process according to any of claims 1 to 6, wherein the temperature at the top of the main column (MC) or of the dividing wall column (DWC) is set within the range from 70 to 130°C, preferably within the range from 85 to 115°C, more preferably within the range from 95 to 105°C, and the temperatures in the bottom of the main column (MC) or of the dividing wall column (DWC) are each set within the range from 150 to 200°C, preferably within the range from 160 to 190°C, more preferably within the range from 170 to 180°C.

8. The process according to any of claims 1 to 7, wherein the distillative purification of crude DMAc is carried out in a column configuration (I) whose main column (MC) has a gaseous sidestream and whose sidestream column (SC) is operated in rectifying mode.

9. The process according to claim 8, wherein the main column (MC) has a smaller diameter above the gaseous sidestream takeoff compared to the region of the main column (MC) below the gaseous sidestream takeoff.

10. The process according to any of claims 1 to 7, wherein the distillative purification is carried out in a column configuration (I) in which the main column (MC) has a liquid sidestream and the sidestream column (SC) is operated in stripping mode.

11. The process according to any of claims 1 to 10, which is operated continuously.

## Revendications

1. Procédé de purification par distillation de diméthylacétamide brut aqueux (DMAc brut), contenant du DMAc, des fractions légères et des fractions lourdes, dans lequel les fractions lourdes et les fractions légères sont séparées avec obtention de DMAc pur dans une des configurations de colonnes spécifiées ci-dessous:
(I) une colonne principale (HK) avec colonne à sortie latérale (SK), ou
(II) une colonne à paroi de séparation (TWK)
**caractérisé en ce que**, dans la configuration de colonnes (I), au moins la colonne principale (HK) et, dans la configuration de colonnes (II), la colonne à paroi de séparation (TWK) sont mises en service avec une pression de tête dans la plage de 0,5 à 1,8 bar absolu.

2. Procédé selon la revendication 1, **caractérisé en ce que**, dans la configuration de colonnes (I), au moins la colonne principale (HK) et, dans la configuration de colonnes (II), la colonne à paroi de séparation (TWK) sont mises en service avec une pression de tête dans la plage de 0,8 à 1,5 bar absolu, de manière préférée dans la plage de 1,0 à 1,3 bar absolu.

3. Procédé selon la revendication 1 ou 2, **caractérisé en ce que** la colonne à sortie latérale (SK), dans la configuration de colonnes (I), est mise en service avec une pression de tête dans la plage de 0,5 à 1,8 bar absolu, de manière préférée de 0,8 à 1,5 bar absolu, de manière particulièrement préférée de 1,0 à 1,3 bar absolu.

4. Procédé selon l'une quelconque des revendications 1 à 3, **caractérisé en ce que** des garnissages à action séparatrice avec des temps de séjour élevés, de manière préférée des plateaux, sont incorporés dans la section de stripping de la colonne principale (HK) dans la configuration de colonnes (I) et/ou dans la section de stripping de la colonne à paroi de séparation (TWK) dans la configuration de colonnes (II).

5. Procédé selon l'une quelconque des revendications 1 à 4, **caractérisé en ce que** 5 à 30, de manière préférée 10 à 25, de manière particulièrement préférée 12 à 18 étages de séparation théoriques sont prévus dans la section de stripping de la colonne principale (HK) ou de la colonne à paroi de séparation (TWK).

6. Procédé selon l'une quelconque des revendications 1 à 5, **caractérisé en ce que** la colonne principale (HK) ou la colonne à paroi de séparation (TWK) est équipée à chaque fois d'un évaporateur de pied et d'un condenseur à la tête de colonne.

7. Procédé selon l'une quelconque des revendications 1 à 6, **caractérisé en ce que** la température à la tête de la colonne principale (HK) ou de la colonne à paroi de séparation (TWK) est ajustée dans la plage de 70 à 130°C, de manière préférée dans la plage de 85 à 115°C, de manière particulièrement préférée dans la plage de 95 à 105°C, les températures dans le pied de la colonne principale (HK) ou de la colonne à paroi de séparation (TWK) sont ajustées à chaque fois dans la plage de 150 à 200°C, de manière préférée dans la plage de 160 à 190°C, de manière particulièrement préférée dans la plage de 170 à 180°C.

8. Procédé selon l'une quelconque des revendications 1 à 7, **caractérisé en ce que** la purification par distillation de DMAc brut est mise en oeuvre dans la configuration de colonnes (I), dont la colonne principale (HK) présente une sortie latérale de gaz, et dont la colonne à sortie latérale (SK) est mise en service selon un procédé de renforcement.

9. Procédé selon la revendication 8, **caractérisé en ce que** la colonne principale (HK) présente au-dessus de la sortie latérale de gaz un diamètre plus faible par rapport au secteur de la colonne principale (HK) situé au-dessous de la sortie latérale de gaz.

10. Procédé selon l'une quelconque des revendications 1 à 7, dans lequel la purification par distillation est mise en oeuvre dans la configuration de colonnes (I), dans laquelle la colonne principale (HK) présente une sortie latérale de liquide, et la colonne à sortie latérale (SK) est mise en service selon un procédé de stripping.

11. Procédé selon l'une quelconque des revendications 1 à 10, **caractérisé en ce qu'**il est mis en oeuvre en continu.
